# EUROPEAN PATENT APPLICATION

(11) **EP 4 328 296 A1**
(43) Date of publication of application: **28.02.2024**
(21) Application number: 22191856.8
(22) Date of filing: 24.08.2022
(51) Int. Cl.: C12M 1/40, A23L 13/40, C12M 1/12, C12M 1/42, C12N 5/00, D01D 5/00, D01F 6/92

(54) **METHOD FOR OPERATING A BIOREACTOR FOR CULTIVATED MEAT AND CORRESPONDING BIOREACTOR**

(71) Applicant: Mirai Foods AG, 8820 Wädenswil (CH)
(72) Inventor: MOREL, Alexander, 4600 Olten (CH); WIRTH, Sylvie Christine, 8805 Richterswil (CH); RAZZA, Nicolò, 8804 Au ZH (CH); HÜBSCHER, Joël, 3292 Busswil (CH); EBERLE, Sebastian, 8820 Wädenswil (CH)
(74) Representative: Bremi, Tobias Hans

(57) **Abstract**

Method for the manufacturing of an elongated, e.g. microfibril structure for the fabrication of cultivated meat, wherein
(a) a paste is extruded through an extrusion plate (24) and an adjacent attachment plate (22) with aligned nozzle openings (42, 44) into a space (58) downstream of the second nozzle plate (22),
(b) paste located in said space (58) or in the attachment plate (22) is hardened,
(c) said paste is continued to be extruded through said extrusion plate (24) while the distance between the attachment plate (22) and the extrusion plate (22) is increased under formation of said elongated, e.g. microfibril structure between said plates (22,24).

## Description

### TECHNICAL FIELD

The present invention relates to a method for manufacturing elongated structures, typically fibrous, preferably microfibrillar structures for making cultivated meat, in particular for making efficiently large bundles of several tens or 100s or even many thousands of such elongated, typically fibrous structures in parallel, as well as to a reactor for implementing such a method most efficiently and reliably. It furthermore relates to corresponding cultured meat products obtained using the method and/or the respective reactor.

### PRIOR ART

Meat is an important protein source of the human diet. Controversial animal welfare associated with the traditional meat industry along with the increasing global population and demand for meat products, sustainable production alternatives are indispensable. Cultivated meat technology provides an opportunity to produce edible sources of animal protein that are not associated with the environmental impact of animal farming. In meat products, aligned fibrous/fibrillar structures are present at the tissular, cellular, and molecular scale levels, and are responsible for texture, tenderness, organoleptic and nutritional characteristics of meat products. In its current status, cultivated meat production is challenged by the availability of scalable and cost-efficient processes to produce tissues with high anisotropic fibrous structuration characteristic of animal meat , and reactors to culture said tissues at very high density and capable of applying cyclic mechanical stimulations.

In order to replicate a multiscale anisotropic structuration in a cultured meat product, it is important to employ a scaffolding technique that produces fibrous/fibrillar structures that guide the muscle progenitor stem cells to maturely differentiate in aligned myofibers. Additionally, the application of cyclic mechanical stretching during culture can promote cell differentiation and anisotropic orientation. Like meat obtained from the flesh of slaughtered animals, cultivated meat products should be produced in very large quantities and to a reasonably low price. For these reasons, not only the scaffolding and cultivation techniques employed are expected to be safe for human and non-human consumption, but they should also be scalable and compatible with high production rates. Keeping these points in mind, prior art available in patents and scientific literature is discussed in the following paragraphs. In WO-A-2020160533 fibrous scaffolds for cultivated meat production are disclosed. The scaffold consists of a porous fibrous mat. Cells are integrated in the product by seeding the scaffold with a cell suspension. The seeding step is the critical one, and it can last hours to days depending on the used cells. The seeding efficiency is controlled by the cell attachment kinetic and by the bioavailability of attachment sites. Low seeding efficiency can be experienced. The seeding is limited by the available surface area, and consequently, the cell density achievable in the final products is limited.

In MacQueen a al. (Muscle tissue engineering in fibrous gelatin: implications for meat analogs. npj Sci Food 3, 20 (2019)) the preparation of fibrous gelatin scaffold is reported by immersion rotary jet spinning at high rates (~ 100 g/h, dry weight) and, depending on process conditions, the approach allows the fabrication of edible scaffolds for cultured meat production in a scalable manner. However, as the scaffold relies on post-production cell seeding, the achievable seeding density is limited by the surface available area as confirmed by the lack of a mature muscle architecture.

In US7,622,299, bioengineered tissue substitutes are reported. The invention reports the fabrication of muscle tissues with a high degree of anisotropy consisting of aligned microfibers array. In this invention, microfibers need to be prepared individually and then arranged in arrays consisting of multiple fibers in parallel stacked in layers. Additionally, cells need to be seeded on the construct. Therefore, the same drawbacks mentioned for other porous scaffolds seeded post-fabrication will also apply here. Additionally, the disclosed invention requires an equipment with a large footprint compared to the amount of produced fibers and is thus not suitable for high scale production.

In Kang et al (Engineered whole cut meat-like tissue by the assembly of cell fibers using tendon-gel integrated bioprinting, NATURE COMMUNICATIONS | (2021) 12:5059), a scaffolding method based on cell-laden gels is reported. The reported method overcome some of the seeding issues mentioned for the above-mentioned prior art. The gel formulation is based on fibrinogen crosslinked by thrombin and embedding cell and collagen microfibers. Because of the composition of such formulation, the sol to gel transition has a slow kinetics and therefore to produce fibers by molding in a sacrificial gel. Because of these reasons, the disclosed method and compositions are hardly scalable to produce a large quantity of cultivated meat.

In Distler et al (3D printed oxidized alginate-gelatin bioink provides guidance for C2C12 muscle precursor cell orientation and differentiation via shear stress during bioprinting, Biofabrication 12 (2020) 045005) a gel paste formulation containing oxidized alginate and gelatin in combination to shear stresses are used to provide guidance to cell orientation and differentiation. The formulation of this prior art allows fast gelation which could be compatible high production capabilities. Nevertheless, bioprinting is slow process used for precisely depositing materials in space, most often from a single nozzle at a time, and is thus not a promising technology for mass production.

In Kessel et al (3D Bioprinting of Macroporous Materials Based on Entangled Hydrogel Microstrands, Adv. Sci. 2020, 7, 2001419) a method to prepare aligned hydrogel fiber bundles is reported. The method is used in a bioprinting setup by depositing the fibers on a substrate or in a molding process setup. Both processes are not scalable for the production and culture of high quantity of biological tissues in sterile conditions.

In Zhang et al (Creating polymer hydrogel microfibres with internal alignment via electrical and mechanical stretching, Biomaterials, Volume 35, Issue 10, March 2014, Pages 3243-3251), authors report a strategy for the generation of hydrogel microfibres with internal alignment induced by a combination of electrical and mechanical stretching. Cells can be loaded directly in the hydrogel formulation cells (i.e., a seeding step is not required). Nevertheless, the process requires the use of high voltage (3000-5000V). The use of high voltage in processing/manufacturing facilities would pose risk for electric arcs and poses specific requirements in terms of electric insulation of machines. In addition to that, the reported process is relatively slow.

WO-A-2019211189 discloses an apparatus for the production of tissue from cells. The apparatus comprises an elongate body having at least one circumferential groove and being operable to extend, by close-fitting relationship, centrally through at least one trough. The troughs are extending in a closed path, such that the at least one of the circumferential grooves open into an inner edge of a trough. Also disclosed is a process for production of tissue from cells, via a transitioning intermediate which transitions from the cells into the tissue.

US20210017485 discloses a cell expansion system for culturing and expanding cells in hydrogel tubes is disclosed herein. The cell expansion system allows for expanding cells that can significantly reduce the production time and cost, while increase the production capacity.

Hoesli et al (A novel alginate hollow fiber bioreactor process for cellular therapy applications in Cell Culture and Tissue Engineering, Vol. 25 (6), p. 1740-1751) report that gel-matrix culture environments provide tissue engineering scaffolds and cues that guide cell differentiation. For many cellular therapy applications such as for the production of islet-like clusters to treat Type 1 diabetes, the need for large-scale production is anticipated. The throughput of the commonly used nozzle-based devices for cell encapsulation is limited by the rate of droplet formation to ~0.5 L/h. This work describes a novel process for larger-scale batch immobilization of mammalian cells in alginate-filled hollow fiber bioreactors

(AHFBRs). A methodology was developed whereby (1) alginate obstruction of the intra-capillary space medium flow was negligible, (2) extra-capillary alginate gelling was complete and (3) 83 ± 4% of the cells seeded and immobilized were recovered from the bioreactor. Chinese hamster ovary (CHO) cells were used as a model aggregate-forming cell line that grew from mostly single cells to pancreatic islet-sized spheroids in 8 days of AH FBR culture. CHO cell growth and metabolic rates in the AHFBR were comparable to small-scale alginate slab controls. Then, the process was applied successfully to the culture of primary neonatal pancreatic porcine cells, without significant differences in cell viability compared with slab controls. As expected, alginate-immobilized culture in the AHFBR increased the insulin content of these cells compared with suspension culture. The AHFBR process could be refined by adding matrix components or adapted to other reversible gels and cell types, providing a practical means for gel-matrix assisted cultures for cellular therapy.

No part of this discussion of the prior art is to be construed as an admittance of prior art.

### SUMMARY OF THE INVENTION

Indeed, such multiscale anisotropic elongated structures characteristic of animal meat made of muscle bundles (e.g., group of aligned muscle fascicles, myofibers and myofibrils) can only be achieved by producing scaffolds mimicking the aligned elongated, typically fibrous structure of meat and guiding the cells, normally stem cells, differentiation process with the help of a suitable microenvironment, as well as topological and mechanical cues. Particularly good scaffolds for the growth of muscle cells incorporate anisotropic microstructures, in the sense that they have an internal material structure with a preferred direction along the axis. The problem with the provision of such structures is that it requires demanding manufacturing processes under sterile conditions that restricts the process scalability or increases the equipment footprint.

In this invention, we disclose a method and compositions allowing the production of elongated structures, in particular of fibrous meat bundles with multiscale structural characteristics like those of animal meat, and reactors to cultivate said elongated structures, in particular fibre bundles, at high density and capable of applying mechanical stimulations. Such a reactor is designed for the cell seeding and to cultivate e.g. porous fibrous bundles or mats at high density, while optionally applying mechanical stretching.

The disclosed invention comprises a method and reactors for the in-vitro production of elongated structures, in particular of meat tissue or precursors thereof for cultured meat production allowing inter alia the scalable production of muscle bundles of high quality and in large quantities.

More specifically, the invention discloses (i) a method for the fabrication and optionally also the cultivation of fibrous cell-laden gel structures, (ii) a reactor for carrying out corresponding method and (iii) products obtained using the method/reactor. The elongated, in particular fibrous structures support cell differentiation e.g. into muscle bundles with fibrillar and highly anisotropic texture.

According to a first aspect of the present invention it therefore relates to a method for the manufacturing of an elongated structure, preferably fibrous structure (including microfibril structures) for the fabrication of cultivated meat, preferably for the parallel manufacturing of a multitude of such elongated, preferably fibrous structures or a bundle of such elongated, preferably fibrous structures.

The method is characterised by the steps as defined in claim 1.

More specifically, according to the proposed method,
(a) a paste is extruded through an extrusion plate with at least one nozzle opening (including round but also elongated openings or specifically shaped openings or a combination of differently shaped openings) and at least partly through and/or or into (into without fully through to extent at least sufficient for attachment after hardening for the drawing process) an essentially adjacent attachment plate (preferably with aligned nozzle openings), preferably into a space downstream of attachment plate, wherein preferably just enough paste is extruded through the two plates such that in or behind the attachment plate the front part is penetrating into the attachment plate, e.g. in that either a layer of the paste is formed or at least in or behind each nozzle opening or porosity of the attachment plate the paste is located and/or is flowing apart to a diameter larger than the nozzle opening,
(b) paste located in said attachment plate and/or in said space downstream of attachment plate is at least partly hardened, leading to a quasi-automatic fixing or attachment of the paste in and/or behind of the attachment plate,
(c) said paste is continued to be extruded through said extrusion plate while the distance between said extrusion plate and said attachment plate is continuously increased under formation of said elongated structure between said plates.

In other words in the final step (c) extrusion runs in parallel with the distancing of the plate from each other such that in a controlled manner the elongated structure or the bundle of elongated structures in case of a plurality of nozzle openings is generated.

Preferably, the distancing speed of the attachment plate from the extrusion plate is larger than the extrusion speed, so there is a certain degree of drawing of the elongated structure during extrusion.

Correspondingly according to a first preferred embodiment, the extrusion takes place at an ejection speed under the generation of a speed gradient downstream of the respective nozzle opening in the extrusion plate by way of a distancing speed between said extrusion plate and said attachment plate larger than the ejection speed to form said elongated structure. The ejection speed is defined as the past (e.g. hydrogel) input flow rate divided by the total nozzle opening area.

In step (c) the pulling rate, measured as the speed of the attachment plate relative to the extrusion plate (the relative speed is relevant, the extrusion plate can be stationary or attachment plate can be stationary or both can be moving relative to each other), is preferably in the range of 0.01-100 m/min, more preferably between 0.1-10 m/min.

To make sure that the above-mentioned attachment of the paste downstream of attachment plate is established, according to a further preferred embodiment in at least one of: step (b), step (c) and after step (c), the drawn/extruded paste is immersed in a hardening bath, preferably a hardening bath comprising divalent or polyvalent cations or an acidic bath or a bath with a temperature different from the gel paste, more preferably a hardening bath comprising divalent cations.

Said extrusion plate and/or said attachment plate preferably each comprise a plurality of nozzle openings, preferably at least 20, more preferably at least 100, most preferably at least 200 or in the range of 250-1000 nozzle openings,
and/or the nozzle openings cover 5 - 90% or between 5% and 95% of the plate cross-section area, more preferably between 20% and 90% or 20 - 80%, most preferably between 40% and 70%,
and/or the nozzle openings are provided in said extrusion plate and/or said attachment plate with a nozzle density of 1 - 5000 nozzles/cm2, preferably in the range of 5 - 400 nozzles/cm2.

Preferably the nozzle openings have a diameter or maximum extension in the lateral direction in case of non-circular openings in the range of between 10 to 5000 µm, more preferably 100-1500 µm, in particular the above nozzle specifications in terms of numbers, % and/or densities are given in combination with nozzle sizes with nozzle openings having a diameter or maximum extension in the lateral direction in case of non-circular openings in the range of between 10 to 5000 µm, more preferably 100-1500 µm.

The nozzle openings can be of circular cross section, oval, rectangular, square-shaped opening, and most preferably all with the same circular diameter, which are preferably arranged in an overlapping manner in the two plates, if also the attachment plate is provided with nozzle openings.

The attachment plate can be a mesh, a porous plate, or a plate with nozzle openings, preferably a plate with nozzle openings aligned to nozzle openings of the extrusion plate. If it is a mesh, a porous plate, the structure of the porosity has to be such that partial penetration of the paste which is extruded through the extrusion plate into the attachment plate is possible, and that hardening of that paste in porosity of the attachment plate is possible, for example by flooding the space downstream of the attachment plate with a hardening agent or by irradiation. Like that it is for example possible to work in situations where the paste only partly penetrates into the attachment plate into the porosity without fully penetrating the attachment plate, and then the paste is hardened in that porosity in the attachment plate leading to an attachment of the strings of paste for the subsequent extrusion process.

As concerns the nozzle openings in the extrusion plate, and if the attachment plate comprises corresponding nozzle openings also and in the same way as these nozzles, these nozzle openings can be arranged in a density and pattern adapted to the desired bundle structure. It is for example possible in particular in case of larger extrusion/attachment plates, to have separate areas with nozzle openings suitable and adapted to form fibre bundles which correspond to a desired shape of the meat product, for example a string of artificial muscle, while having areas between these nozzle opening areas without nozzle openings, so that in one process several such fibre bundles similar to natural muscle strings are generated and, if needed and desired, directly subsequently cultivated. The above-mentioned densities and percentages preferably apply specifically only to such areas of nozzle openings.

Preferably, the nozzle openings have a diameter or maximum extension in the lateral direction in case of non-circular openings in the range of between 10 to 5000 µm, more preferably 50-1000 µm.

Preferably the nozzle openings are of circular form and most preferably all with the same circular diameter, which are arranged in an overlapping manner in the two nozzle plates.

According to yet another preferred embodiment, extrusion in steps (b) and (c) takes place into a preferably closed sterile reaction container (bioreactor, cultivation vessel), and subsequent to step (c), if needed followed by a step of further hardening of the extruded elongated structure, the reaction container is filled with culturing growth media and the elongated structures are used for growing meat cells seeded onto said structures and/or already contained in said paste.

According to a particularly preferred embodiment, the proposed method is characterised in that
(a) said paste is extruded through said extrusion plate and at least partly through an adjacent attachment plate with aligned nozzle openings, preferably into a space downstream of the attachment plate,
(b) slowing down or stopping said extrusion and hardening paste located in said attachment plate and/or in said space by flooding said space downstream of the attachment plate with a hardening bath, preferably a hardening bath comprising divalent or polyvalent cations or an acidic bath, or a bath with a temperature different from the gel paste, preferably a bath of calcium chloride,
(c) followed, if need be after removal of the hardening bath, or in the presence of the hardening bath, by continued extrusion of said paste through said extrusion plate while distancing said attachment plate with a relative distancing speed larger than the extrusion speed from said extrusion plate under formation of said elongated structure, preferably of a multiscale anisotropic structure between said nozzle openings,
(d) slowing down or stopping said distancing and extrusion, and hardening the paste in the form of microfibrils in the space between the two plates, by flooding said space downstream of the attachment nozzle plate with a hardening bath if not done so already during step (c), preferably a hardening bath comprising divalent or polyvalent cations or an acidic bath or a protein cross-linking bath, preferably a bath of calcium chloride, and, optionally, hardening the paste in a space upstream of the extrusion plate,
(e) replacing what is in the space between the plates (typically the hardening bath or the cross-linking bath) with culturing media and using the elongated structures, preferably fibre bundles (e.g. microfibrils) for growing meat cells seeded onto said elongated structures and/or already contained in said paste.

The corresponding process can be controlled by controlling the composition of the media, the temperature, pH, the supernatant gas atmosphere, et cetera.

According to a further preferred embodiment, during culturing the two plates can be oscillated relative to each other stimulating and influencing the growth process in the anisotropic microfibrillar structures.

Preferably, said paste is a paste for the fabrication of cultivated meat, comprising of consisting of the following components:
(A) at least one polysaccharide that can form a solidified gel by the action of divalent or polyvalent cations, thermal gelling, light-induced addition reaction or light induced condensation reaction, or a combination thereof, preferably in a concentration in the paste in the range of 0.01-200 g per L of component (D);
(B) preferably at least one protein, preferably assembling with the polysaccharide of component (A) via supramolecular or covalent interaction or a combination thereof, preferably in a concentration in the paste in the range of 0.001-500 g per L of component (D);
(C) cells selected from mammalian cells, fish cells, crustaceous cells or a combination thereof, in a concentration in the paste in the range of 0-300 billion cells per L of component (D);
(D) water or a water-based culturing medium;
(E) additives different from (A)-(D), preferably selected from the group consisting of crosslinking kinetic modifier in a concentration in the paste in the range of 0-500 mM, flow modifier in a concentration in the paste in the range of 0-200 g per L of component (D), or a combination thereof.

In fact, preferably a paste as described in European application EP 22 164 793.6 is preferably used in this method. The disclosure of European application EP 22 164 793.6 is included into the specification as concerns the paste and methods of treating the paste as well as corresponding products as defined in that European application.

The major features of this paste and its use in the method are described below:
(i) The gel paste composition in combination with the method of production allows the fabrication of cell-laden composite gels characterized by unique anisotropic elongated, preferably fibrillar structure at the microscale.
(ii) The production process to develop such elongated, preferably fibrillar structure is fast and scalable and therefore compatible with the high production rate required for cultivated meat production.
(iii) The elongated, preferably fibrillar structure of the composite gel promotes a superior differentiation of muscle progenitor stem cells into muscle tissue with the features typical of meat.

Advantages resulting from the major features of the invention comprise the following aspects, alone or in combination:
(1) Gel paste is composed of edible components for the formation of the microfibrillar structure.
(2) Differentiated cells assemble in muscle bundles with realistic size. Muscle bundles have a tunable diameter within 0.01 mm and 2 mm and a length tunable in size above 1 mm.
(3) Size of the muscle bundle can be tuned by playing on different process parameters. This allows the production of muscle fibers with a texture that mimics that of meat of different species.
(4) High cell density can be achieved, comparable to animal muscle tissue. Cell density up to hundreds of million cells per cm³ can be achieved
(5) Cell seeding is efficient (with virtually 100% seeding efficiency) and homogeneous throughout the whole scaffold volume
(6) Gel paste composition and process parameters can be tuned to promote cell spreading and differentiation by fulfilling biomechanical requirements of any specific cell type.
(7) Fibrous gels embedding cells withstand culturing conditions for several weeks.
(8) Gel paste is converted into a mechanically stable gel within hundreds of seconds with cells being trapped and protected inside, making gel fibers easy to handle and compatible with a high production rate. Muscle bundles production can be completely automated, and production can happen directly inside the differentiation bioreactor.
(9) As a consequence of the fibrous texture of the gel entrapping cells, the resulting muscle bundles develop superior aligned fibrillar structure even without active tension during culture.
(10) High increase of protein content is observed upon differentiation as a consequence of the highly anisotropic gel microstructure and cell differentiation
(11) Mild processing conditions
(12) Optionally, muscle bundles are physically clamped during production in order to straightforwardly apply external stimuli (e.g. mechanical stretching and/or electrical field) to further push myofibrillar/myoglobin protein production.
(13) Optionally, non-proteinaceous components used as processing aids in the preparation of the gel paste (e.g., polysaccharide components) can be degraded/dissolved at the end of the differentiation process to maximize of the protein content and minimize the ingredient list.
(14) Optionally, bioactive molecule can be immobilized in the gel to support differentiation and spreading.
(15) Optionally, chromophores, flavour and aroma molecules can be added to the gel paste to improve overall sensory attributed of the produced meat.
(16) Optionally, fibroblasts can be seed on and/or in the muscle bundle to deposit connective tissue and further tune the texture of the final meat product
(17) Optionally, the crosslinking bath used for the gelation of the gel paste can contain iron ions to increase the iron content of the final meat product.
(18) Optionally, post-cultivation, muscle bundles can be glued together by using microbial transglutaminase. Furthermore, plant proteins and animal proteins can be added to tailor texture and protein content/source.
(19) Optionally, the produced muscle bundle, can be combined with plant, animal and cultivated fat to produce meat-based foodstuffs.
(20) Optionally, micro/nano fibres can be added in the gel paste formulation to increase the toughen the hydrogel and modulate texture.

So the present invention discloses methods and compositions for the fabrication of a cell-laden composite gel which can be formed on-demand with fibrillar structure. The invention is composed of different elements: a composition for a gel paste, and a method to produce gel fibers from the gel paste and the use of it to produce cultivated meat products. According to a preferred embodiment of such a paste, the at least one polysaccharide of component (A) is naturally crosslinkable and/or chemically modified to promote crosslinking, wherein preferably the chemical modifications include acrylation, methacrylation, epoxidation, allylation, or a combination thereof.

According to a further preferred embodiment of such a paste, the at least one polysaccharide of component (A) is selected from the group consisting of alginates, pectins, carrageenans, chondroitin sulfate, dermatan sulfate, heparin, heparin sulfate, as well as derivatives thereof and combinations thereof, preferably the polysaccharide component of component (A) is at least one alginate or alginate derivative.

Preferably, the polysaccharide(s) of component (A) is present in a concentration in the paste in the range of 0.1-100 g per L of component (D).

According to yet another preferred embodiment the at least one protein of component (B) takes the form of a native protein, denatured protein, protein hydrolysate, or a combination thereof.

Preferably the at least one protein of component (B) is bioactive with the ability to modulate a function and/or characteristic of the cells of component (C), in particular to promote cell attachment by providing integrin binding motifs.

The at least one protein of component (B) can be preferably selected from the group consisting of gelatin, collagen, fibrinogen, fibrin, fibronectin, fibroin, elastin, laminin, basic albumins from plant, preferably napin, wherein preferably the protein component (B) is gelatine, more preferably gelatine, type A.

Preferably, the at least one protein of component (B) is chemically modified to promote crosslinking, wherein preferably the chemical modifications include acrylation, methacrylation, epoxidation, allylation, or a combination thereof.

According to a preferred embodiment, the at least one protein of component (B) is crosslinked, preferably by transglutaminase, peroxidase, laccase, tyrosinase, lysyl oxidase, glutaraldehyde, genipin, citric acid, photopolymerization or a combination thereof, preferably by transglutaminase is used, and wherein if transglutaminase is used as crosslinker, it is comprised in an amount of 0-50 U per g of protein, more preferably 0.1-10 U per g of protein.

Typically, the protein(s) of component (B) is present in a concentration in the paste in the range of 0.1-250 g per L of component (D).

According to yet another preferred embodiment, the cells of component (C) are present in a concentration in the paste in the range of 5-100 billion cells per L of component (D). The additives of component (E) preferably include at least one crosslinking kinetic modifier to slow down the gelation kinetics of the crosslinkable polysaccharide by sequestering divalent or polyvalent cations.

Preferably the additives of component (E) include at least one food-compatible compound selected from the group consisting of disodium phosphate, dipotassium phosphate, magnesium phosphate, ethylenediaminetetraacetic acid, ethylenediaminetetraacetic salts, or a combination thereof.

Typically the additives of component (E) are present in a concentration 1-100 mM.

The additives of component (E) May include at least one flow modifier to impart a shear thinning behaviour to the gel past so that the higher viscosity in resting condition mitigate cell sedimentation, whereas the lower viscosity during ejection allows processability, wherein preferably said flow modifiers are edible fillers in the form of micro/nanofibers, preferably insoluble in culturing conditions, and are preferably composed of protein and/or polysaccharide different from the other components of the paste, wherein such edible filler is preferably present at a concentration of 0-200g per L of paste, and wherein further preferably edible polymers as flow modifier can be selected among gums, including gellan gums, guar gums, xanthan gums), PEGs, or combinations thereof, and wherein preferably edible polymers are present at a concentration of 0-50g per L of gel paste.

Preferably, such a paste is further supplemented with a micro-structuring agent, for unfolding of protein structure, preferably selected from the group consisting of bicarbonate, including sodium bicarbonate, potassium bicarbonate, ammonium bicarbonate, magnesium bicarbonate, or a combination thereof. Preferably the micro-structuring agent is added to the paste in an amount to lead to at a concentration ranging from 0.1-300 g per L of paste, preferably, of 1-200 g per L of paste. The micro-structuring agent is preferably added in powder form. The micro-structuring agent and the paste may also take the form of a kit-of parts, which is joined right before carrying out the method for making the microfibril structure as detailed below.

Preferably, the hardening bath comprises Ca2+, Mg2+, Fe2+ and Fe3+ or a combination thereof in water, preferably at a concentration in the range of 1-500 mM, more preferably between 20-100 mM.

The hardening bath of step (c) can be buffered at a pH ranging from 6 to 8, preferably by using HEPES buffer, wherein further preferably the HEPES buffer is used in a concentration comprised between 1-100mM.

Preferably, the paste is extruded from a nozzle preferably with a circular geometry at the ejection point.

According to yet another preferred embodiment, either the hardening bath comprises a protein cross-linking agent or subsequent to step (b) and/or during step (c) the fibres are immersed in a protein cross-linking bath with such a cross-linking agent.

Preferably such a cross-linking agent is selected from the group consisting of transglutaminase, peroxidase, laccase, tyrosinase, lysyl oxidase, glutaraldehyde, genipin, citric acid, or a combination thereof, preferably transglutaminase, and wherein if transglutaminase is used, it is comprised in an amount of 1-2000 U per mL of crosslinking bath.

Further preferably cross-linking is performed at a temperature in the range of 20-45°C, preferably for a time span in the range of 10-120 min, more preferably 30-90 min.

Muscle bundles can be prepared by cultivation of cell-laden microfibrillated hydrogel fibers in an environment promoting myogenesis.

Preferably, the resulting fibres, preferably having an individual diameter in the range of 0.1-50 µm, preferably aligned in the same axis ± 40° with respect of the fiber axis, are converted into muscle tissue by culturing them, e.g. in a step (e) as defined above, in differentiation media designed for the used cell type, wherein if the fibres have been produced from a paste without cells, before culturing the fibres are seeded with cells. Preferably the fibers, e.g. in the form of bundles, are cross-linked together to obtain a solid edible structure, wherein cross-linking of the fibres can be effected by one or several cross-linking agents selected from the group consisting of transglutaminase, peroxidase, laccase, tyrosinase, lysyl oxidase, glutaraldehyde, genipin, citric acid, or a combination thereof, preferably transglutaminase

Normally, the resulting muscle tissue or assembly of is mixed with further constituent to form a consumer cultured meat product.

According to yet another preferred embodiment, the extrusion in step (c) takes place with a drawing factor, defined as the ratio of the ejection speed to the distancing speed of the second nozzle plate relative to the first nozzle plate during step (c), of at least 1.1, preferably at least 1.5 or at least 2.

According to yet another aspect, the present invention relates to a reactor for carrying out the method according as described above, wherein this reactor, which preferably allows to work under sterile conditions, comprises a closable reactor container of elongated shape along a main axis with constant cross-section, preferably of cylindrical or rectangular/square or hexagonal shape, a (preferably stationary) extrusion plate (having the corresponding cross-sectional shape of the reactor container) at one end of the reactor and at least one attachment plate (also having the corresponding cross-sectional shape of the reactor container), preferably movably mounted in said reactor container, elements for controlled supply of paste for extrusion thereof upstream of said extrusion plate and elements for controllably moving said attachment plate along the main axis, as well as means for supplying at least one of culturing media, hardening media, cross-linking media to and from the inside of the reactor container, preferably also to a space upstream of said (stationary) extrusion plate.

According to a preferred embodiment of this reactor, the extrusion plate is located at a bottom of said closable reactor container, and said attachment plate is located above of the extrusion plate.

According to yet another preferred embodiment, the extrusion plate can be moved by said elements in a contactless manner, preferably by magnetic attraction, wherein preferably to this end the extrusion plate is mounted in and/or on a mounting structure with at least one magnet and outside of the reactor container there is a movable magnetic element, preferably in the form of a ring with at least one magnet, which movable magnetic element can be moved automatically, preferably by motor.

Said (stationary) extrusion plate and/or said attachment plate preferably each comprise a plurality of nozzle openings, preferably at least 20, more preferably at least 100, most preferably at least 200 or in the range of 250-1000 nozzle openings,
and/or the nozzle openings cover 5 - 90% or between 5% and 95% of the plate cross-section area, more preferably between 20% and 90% or 20 - 80%, most preferably between 40% and 70%,
and/or the nozzle openings are provided in said extrusion plate and/or said attachment plate with a nozzle density of 1 - 5000 nozzles/cm2, preferably in the range of 5 - 400 nozzles/cm2.

Preferably the nozzle openings have a diameter or maximum extension in the lateral direction in case of non-circular openings in the range of between 10 to 5000 µm, more preferably 100-1500 µm, in particular the above nozzle specifications in terms of numbers, % and/or densities are given in combination with nozzle sizes with nozzle openings having a diameter or maximum extension in the lateral direction in case of non-circular openings in the range of between 10 to 5000 µm, more preferably 100-1500 µm.

The nozzle openings can be of circular cross section, oval, rectangular, square-shaped opening, and most preferably all with the same circular diameter, which are preferably arranged in an overlapping manner in the two plates, if also the attachment plate is provided with nozzle openings.

Preferably the nozzle openings are of circular form and most preferably all with the same circular diameter, which are arranged in an overlapping manner in the two nozzle plates.

Further preferably, said (stationary) extrusion plate and/or said attachment plate further comprises at least one opening for controlled supply of liquids through the respective plate. According to yet another preferred embodiment of the reactor, it is equipped with a control, in particular for controlling at least one of the extrusion speed of the paste, the distancing speed of the attachment plate, the supply of and removal of at least one of culturing media, cross-linking media, hardening media.

According to another aspect of the present invention, it relates to the use of a method as described above for the manufacturing of a consumer cultured meat product. Preferably this entails using the corresponding elongated structures, preferably after cultivation in the form of bundles, in combination with
(i) fat and or oil-based components including cultured fat, vegetable fat or animal fat based components and its derivatives, in particular in combination with separately cultured fat cells and/or separately cultured fat cell aggregates,
(ii) and/or structuring agents including hydrocolloids, and/or cellulose and its derivatives, and/or proteins derived from plant, animal, recombinant technology, cell cultivation;
(iii) and/or connective tissue components including animal derived connective tissue, cultured connective tissue, in particular in combination with cultured fibroblast and/or chondrocytes and/or separately cultured fibroblast and/or chondrocytes aggregates.

Said components can be assembled to form fibre bundles compact bundle, like meat pieces.

Furthermore, the present invention relates to a cultured meat obtained using a method as described above, and/or obtained using a reactor as described above.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a bioreactor according to a first embodiment with a low dead volume, wherein in a) a perspective representation with the upper extrusion plate in a medium lower position is shown, in b) a first axial cut is shown in a plane through the closure mechanism and in c) a second axial cut in a plane perpendicular to the ones given in b);
- Fig. 2: shows a bioreactor according to a second embodiment with a high dead volume, wherein in a) a perspective representation with the upper extrusion plate in a medium lower position is shown, in b) a first axial cut is shown in a plane through the closure mechanism and in c) a second axial cut in a plane perpendicular to the ones given in b);
- Fig. 3: shows an axial cut through the bioreactor according to the first embodiment in a position in which the upper extrusion plate is in the uppermost position and including the mounting of the reactor;
- Fig. 4: shows an axial cut through the bioreactor according to the second embodiment in a position in which the upper extrusion plate is in the lowermost position;
- Fig. 5: shows a perspective view onto a bioreactor according to the first embodiment in a perspective representation including the mounting of the reactor and further elements of the automatic system, wherein the upper extrusion plate is in an medium position;
- Fig. 6: shows the bottom extrusion plate wherein in a) a perspective representation is given, in b) the cut along A-A in c) and in c) a top view;
- Fig. 7: shows the upper extrusion plate, wherein in a) a perspective view from the top is given, in b) a cut along A-A- in d), in c) a perspective view from the bottom and in d) a top view is given;
- Fig. 8: the sequence of operation of the proposed bioreactor, wherein in a) the step of pre-filling the gel is illustrated, in b) the upper clamping is illustrated, in c) the extrusion is illustrated, in d) the lower clamping is illustrated, in e) the cross-linking fill-up is illustrated, in f) the actual cross-linking is illustrated, in g) the filling up with media and the cross-linker drain is illustrated, in h) the media fill-up is illustrated and in i) the cultivation is illustrated.

### DESCRIPTION OF PREFERRED EMBODIMENTS

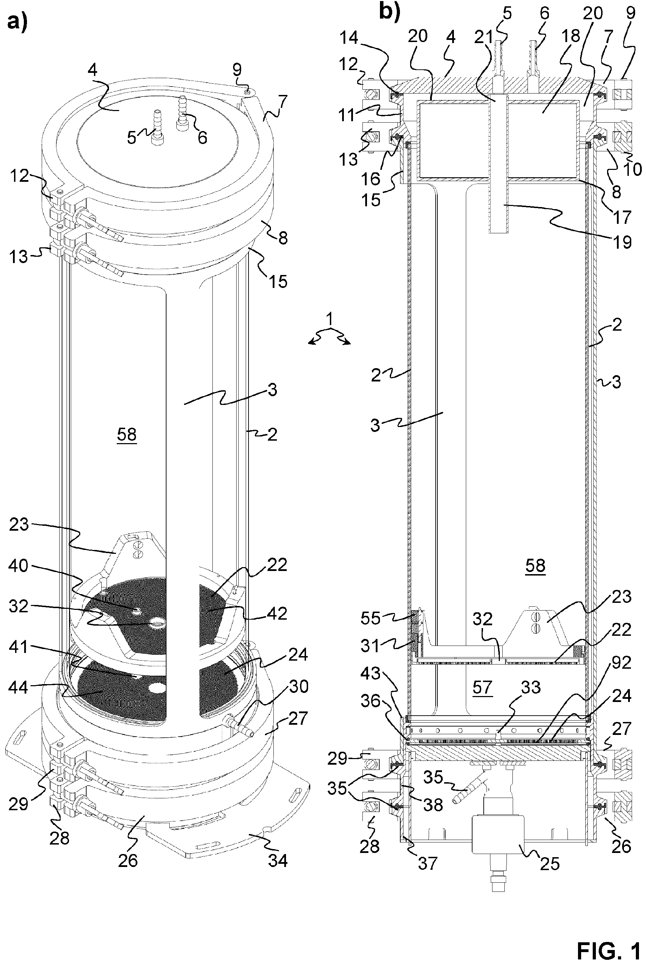

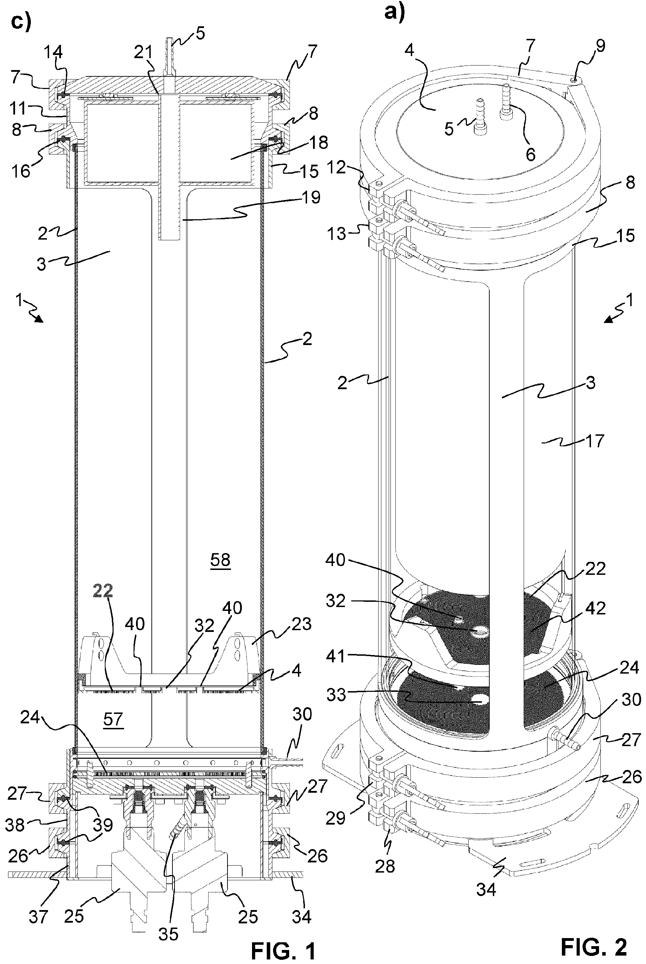

**Fig. 1** shows a bioreactor 1 according to a first embodiment with an insert 17 providing for a low dead volume, i.e. for producing bundles having a high length.

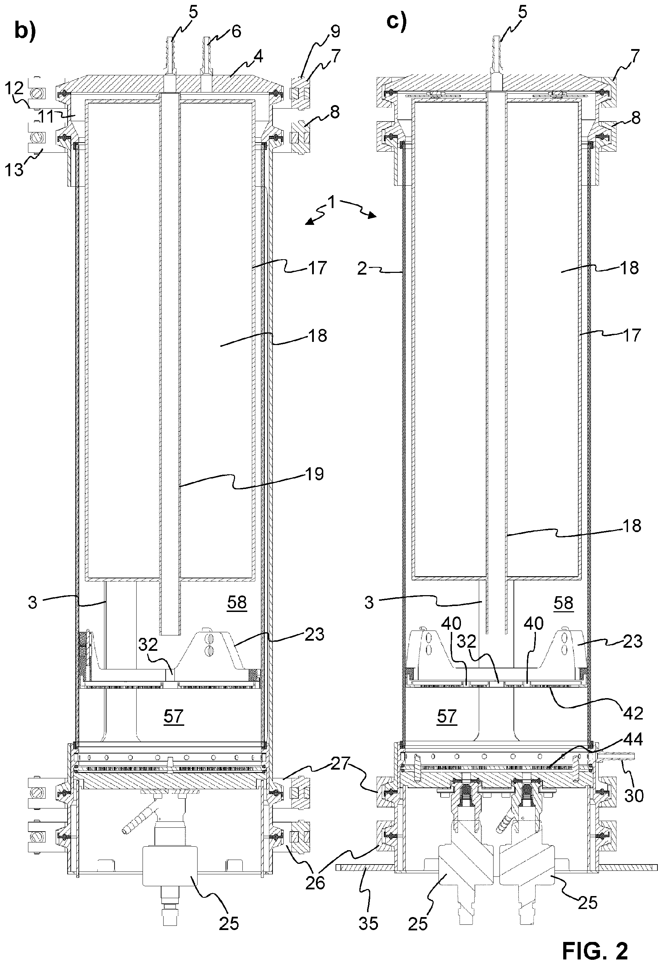

**Fig. 2** on the other hand shows a corresponding bioreactor 1 with a high dead volume, i.e. with an insert 17 to avoid use of too much processing liquids if one desires to produce shorter microfibrillar bundles.

The embodiments according to Fig. 1 and 2 are apart from that essentially the same, and correspondingly the reference numerals in those two figures and in the other ones indicate the same or equivalent elements, in as far as this is not specifically stated otherwise.

As one can see from **Fig. 1a****),** a perspective representation from the top, the reactor 1 comprises a cylindrical circumferential wall 2 which, along its main axis, is supported in the middle section by an axial reinforcement structure 3.

The reactor 1 is covered on the top by a top cover 4, which is provided with a central first inlet/outlet 5, and a second lateral inlet/outlet 6. The top portion of the reactor is based on an upper frame portion 15, which is circumferential and one piece with the axial part 3. That is followed by an upper circumferential closure extension 11 (see Fig. 1b), which is attached to the upper frame portion 15 by way of a lower top closure bracket 8, which hinges around hinge 10 and which is closed by the closure mechanism 13.

Between the upper frame portion 15 and the upper closure extension 11 there is provided a circumferential seal 16. To the top this upper closure extension is followed by the above mentioned top cover 4, which is attached by way of another upper top closure bracket 7, hinged around axis 9, and fixed by closure mechanism 12. Also here there is provided a circumferential sealing 14 between the upper closure extension 11 and the top cover 4. As one can see in particular from the axial cut in **Fig. 1b****) and c)**, in the top portion there is provided an insert 17, which does not have the same but a lower outer diameter than the circumferential wall 2, such that there is an interspace between the insert and the wall, providing for a passage 20 around this insert 17. The insert 17 encloses a dead volume 18, which is not accessible to any liquid. This insert 17 is penetrated by an axial central vertical pipe 19 or channel, which is attached to the first inlet/outlet 5 on top, and sealed relative to that, if needed, by way of a protruding portion 21 in contact with the lower opening of the first inlet 5. On the other hand, the second inlet/outlet 6 is connected with the above-mentioned passage 20 and allows for circulation of liquid through the passage 20 in to the space 58 below the insert.

The reactor as illustrated in **Fig. 1** has an attachment upper plate 22 shown in a lower medium position. This attachment plate 22 is mounted in a circular mounting structure 23, and has a central opening 32, e.g. suitable and adapted to be penetrated or connected/touched with the vertical pipe 19 at its lower end, if the attachment plate 22 has reached the uppermost position. Also there are provided lateral openings 40 in that attachment plate 22. These openings 40 are provided for, if needed, allowing fluid to be supplied and/or removed through the corresponding plate 22. Importantly, this attachment plate 22 is provided with a multitude of nozzle openings or perforations 42, as will be detailed further below.

Below that attachment plate 22, there is located, in a stationary manner, the extrusion plate 24. Also this extrusion plate 24 is provided with a large number of perforations 44, and in fact the perforations in the two plates 22,24 are arranged and the plates are mounted in a way that if the two plates 22,24 are located adjacent to and in flat contact with each other, the perforations in the two plates 22,24 align and allow the extrusion material to pass both plates 22,24, so the nozzle openings in individual plates 22,24 combine to a double plate nozzle opening.

This extrusion plate 24 is provided with surface areas without nozzle openings 41 and 33 aligned with the openings 40 and 32, respectively, in the attachment plate 22. As one can see in particular from **Fig. 1 b)** **and c)**, there can be a material layer 31 around the mounting structure 23 for the attachment plate 22, e.g. to hold the magnets mentioned further below. Also, one can see that in the bottom construction, there is a bottom plate 34, on which there is provided the bottom part 37, which is attached to an upper circumferential bottom part 38, these two elements are attached to each other by way of a lower bottom closure bracket 26, with a corresponding closure mechanism 28. This is followed in the upper direction by the lower frame portion 43, which is attached to the upper bottom part 38 by way of the upper bottom closure bracket 27, with a corresponding closure mechanism 29. Between these elements there is again provided circumferential sealing elements 39.

There is a bottom inlet 25, which allows to supply extrusion material, the above mentioned paste, first to horizontal contiguous space 92 below the first lower extrusion plate 24, this space 92 is also provided with a side inlet/outlet 35. For supply to the space above the extrusion plate 24 and below the attachment plate 22, that is the space 57, there is provided a lateral bottom inlet/outlet 30. The extrusion plate 24 is sealed by way of one or several circumferential sealings 36.

As one can see from the representations in **Fig. 2**, here the insert 17 is much larger in an axial direction and occupies a large fraction of the void volume of the reactor inside the circumferential wall 2. In this sense the space below the insert, that is the space 58, combined with the space 57 between the second upper extrusion plate and the extrusion plate 24, is much smaller. As indicated above, using this insert 17 is suitable and adapted for production processes where short fibrous bundles are to be produced.

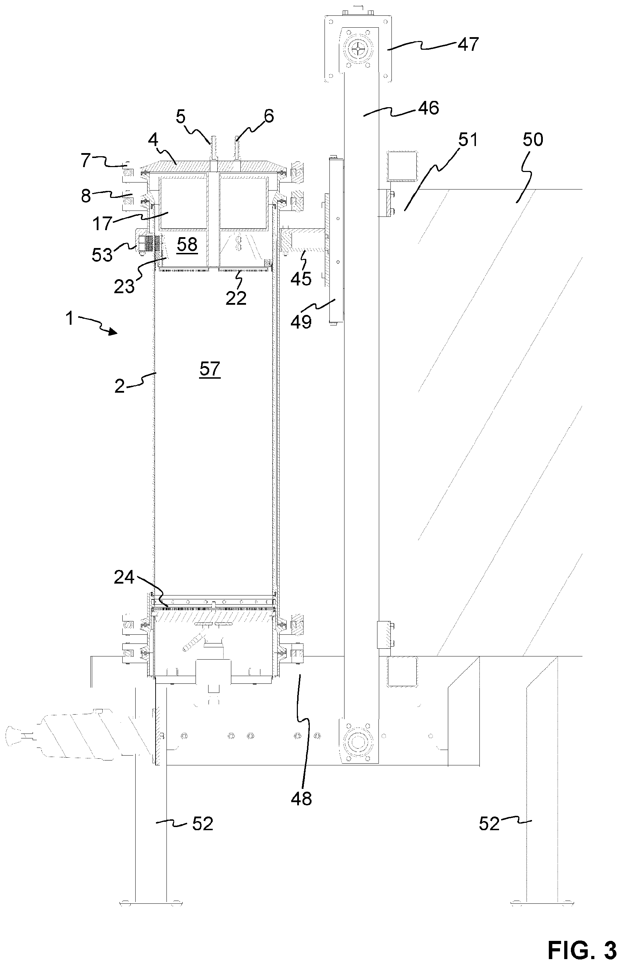

**Fig. 3** shows an axial cut through the bioreactor according the first embodiment (**Fig. 1**) in a position in which the attachment plate 22 is in the uppermost position, and the figure in addition to that shows the mounting of the reactor 1 as well as the means provided for shifting the attachment plate 22 during the extrusion process.

The reactor 1 is mounted on a bottom mounting structure 48, which stands on a number of corresponding vertical legs 52. There is provided a housing 50 which may house or comprise control elements for the movement of the plate 22 or for the supply of the liquid, and a corresponding carrier structure 51. This carrier structure 51 in particular is there to mount a vertical rail 46, on which there is moveably mounted mounting structure 49, on which a shifting bracket 45 is attached. On this shifting bracket 45 there is provided a circumferential shifting ring 53, which controls the axial position of the mounting structure 23 for the attachment plate 22. There is provided a motor 47, and this motor 47, by way of a corresponding belt or chain, allows to vertically move the mounting structure 49 and correspondingly the shifting ring 53 depending on the process.

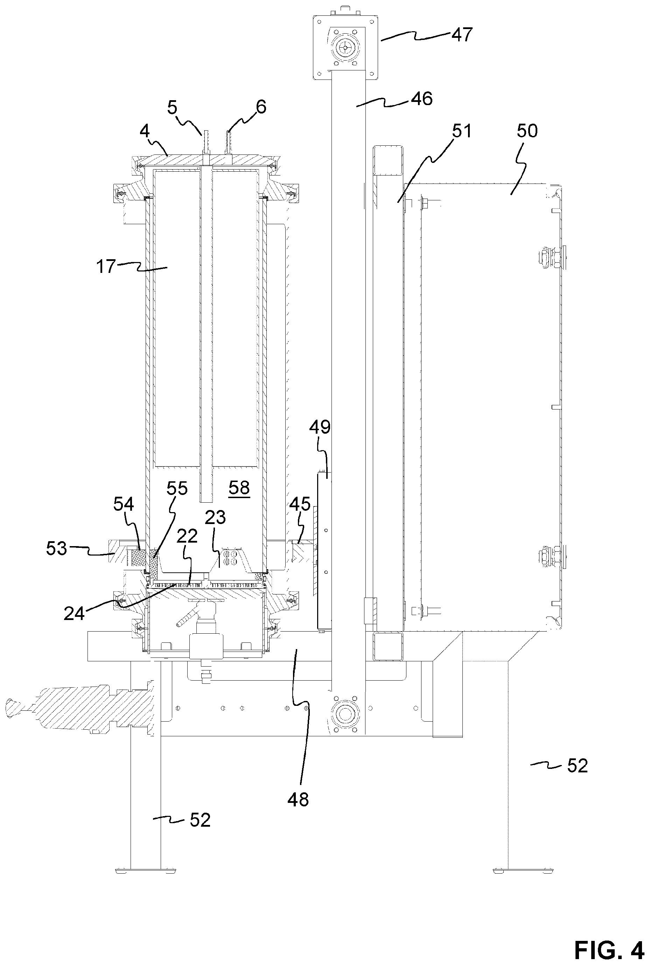

How this is done in a contactless way to allow for sterile conditions in the inside of the reactor 1 is best seen in **Fig. 4**. Here, a corresponding reactor 1 with a large insert, also having a slightly different top cover geometry, so similar to the embodiment in **Fig. 2**, is illustrated in a position where the attachment plate 22 is in the lowermost position, i.e. is adjacent and essentially in contact with the extrusion plate 24. Here one can see, that the shifting ring 53 is provided with magnets 54. Also the carrier structure 23 for the attachment plate 22 is provided, in corresponding axial extensions, with counter magnets 55. Due to the corresponding magnetic attraction, the shifting ring 53 and the mounting structure 23 and the corresponding attachment plate 22 are fixed relative to each other, and if the motor 47 starts pulling the mounting structure 49 in an upwards direction it will correspondingly move the shifting ring 53 upwards and due to the magnetic attraction this will draw the mounting structure 23 and the corresponding attachment plate 22 also in a vertical upwards direction.

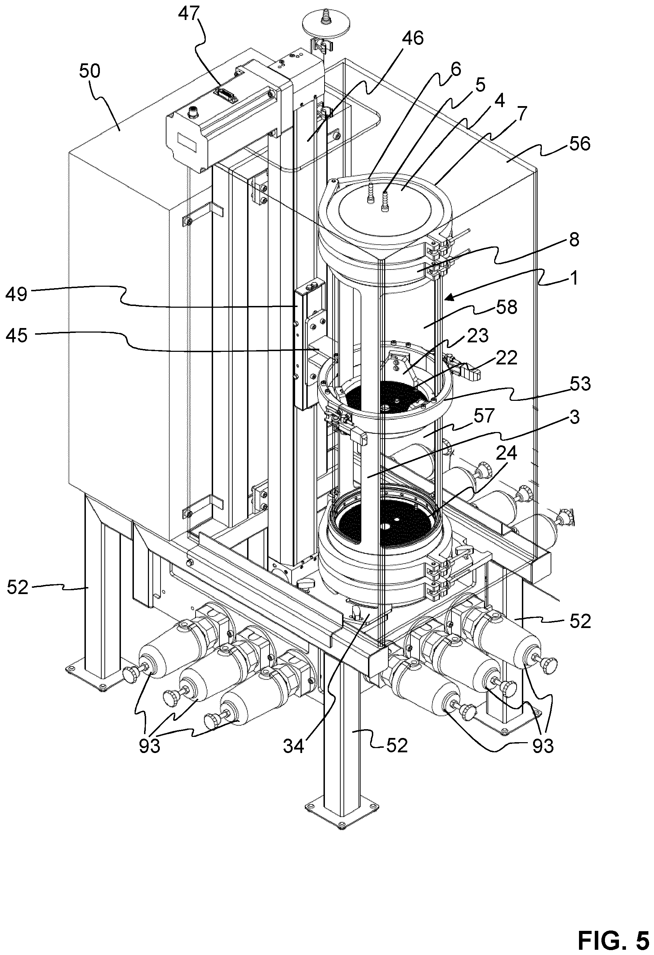

Yet another perspective illustration of the reactor setup with the surrounding elements is illustrated in **Fig. 5**. Here one can see that around the actual reactor there can be provided a glass housing 56, and in the bottom part various control and/or supply handling elements 93 can be provided.

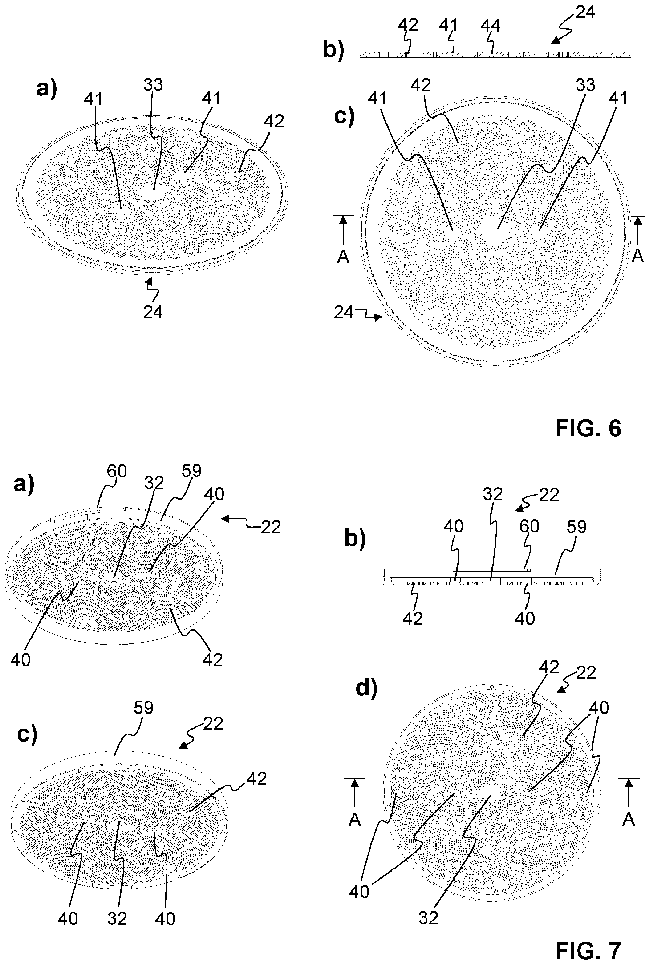

**Fig. 6** illustrates the extrusion plate 24. Here one can see that this is actually a nozzle plate with a very large number of perforations, specifically in this case, there is provided 6700 perforations in this plate. As already indicated above, there is also a first area without openings 33 on the axis of the plate 24, as well as two laterally offset areas without openings 42. The vast majority of the surface of this plate 24 is covered by these openings/nozzles/perforations 42. These perforations have a diameter of 0.7 mm.

**Fig. 7** shows corresponding representations of the second upper extrusion plate. In this case, the plate 22 is provided with a circumferential rim 59, which is provided with an attachment rib 60 for attaching it to the mounting structure 23.

Also, in this case, the central opening 32 is provided with a circumferential rim protruding upwards, and so are the lateral openings 40 as mentioned above.

Again the vast majority of the surface of this plate 24 is covered by the perforations, and the perforations have the same distribution and geometric arrangement as well as the same size as the perforations in the extrusion plate 24 illustrated in **Fig. 6**. This is why, if the two plates 22,24 are put adjacent and in flat contact to each other, these openings will align and form the extrusion nozzles in the first phase of the making process.

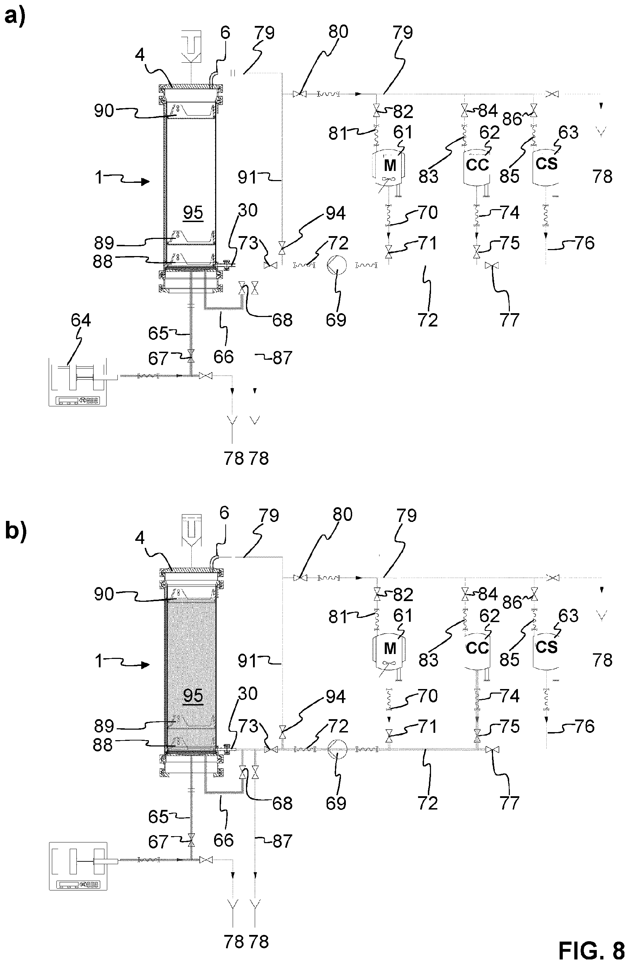

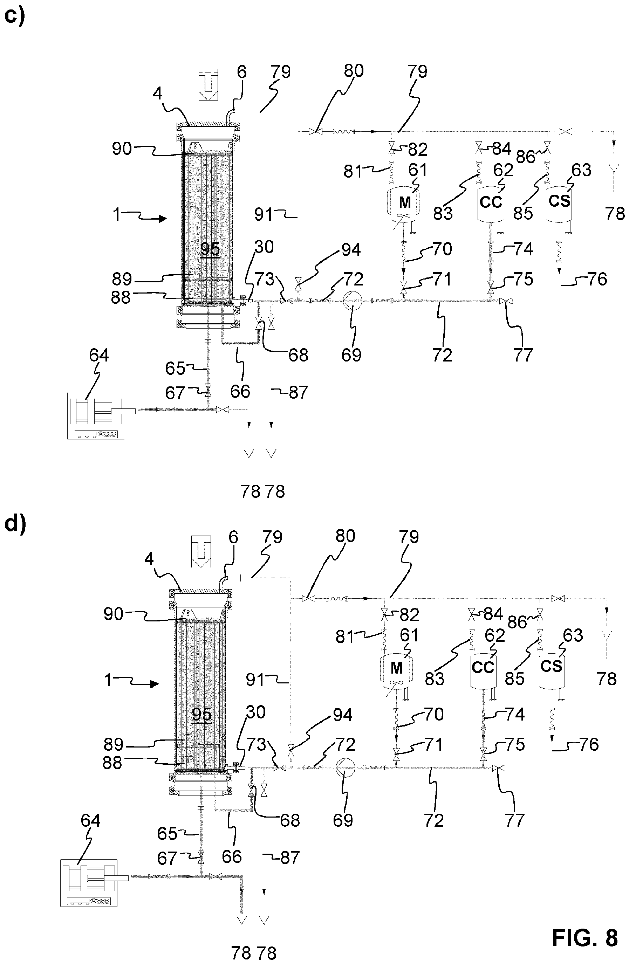

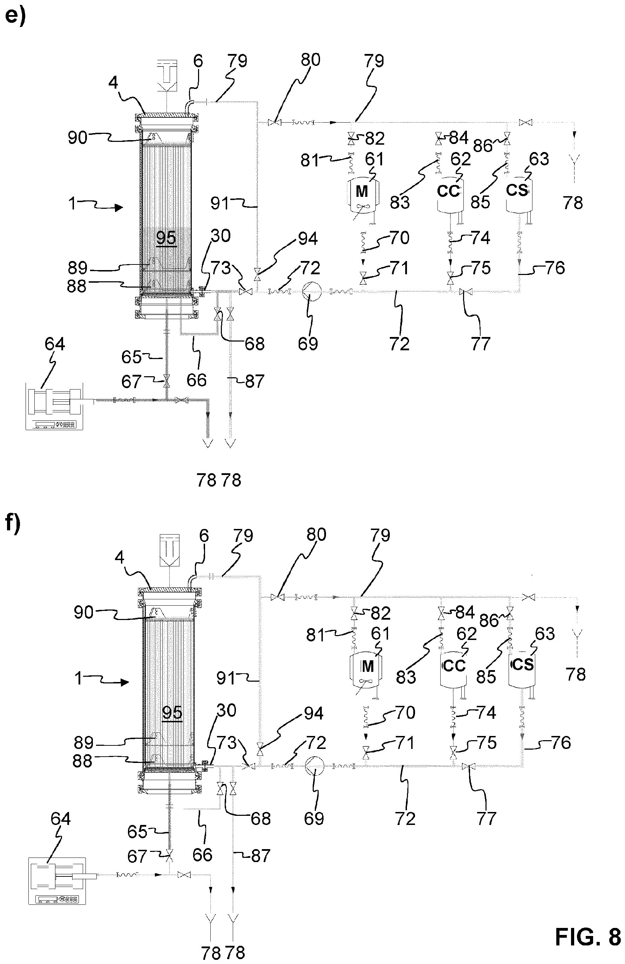

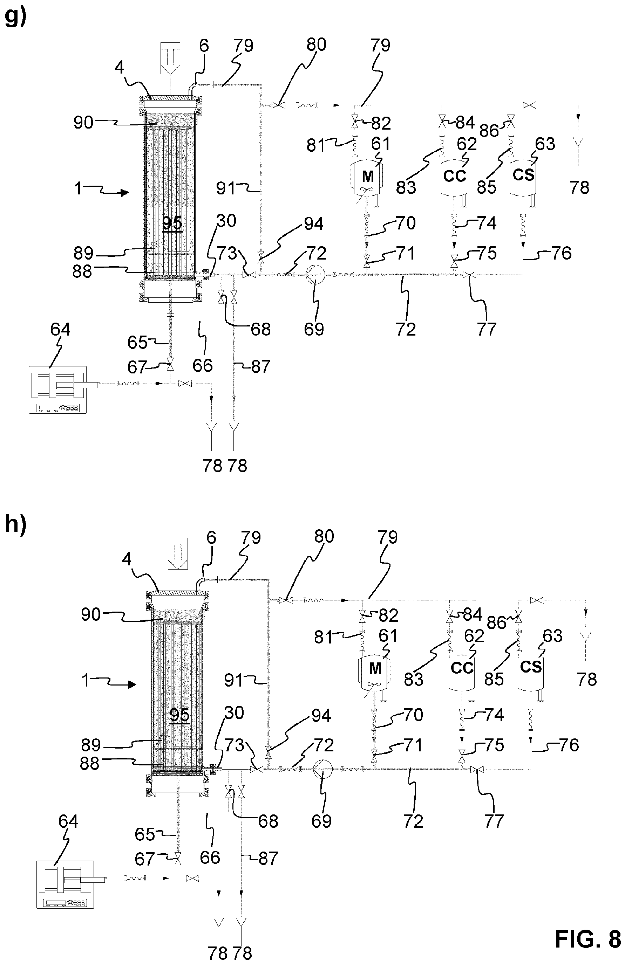

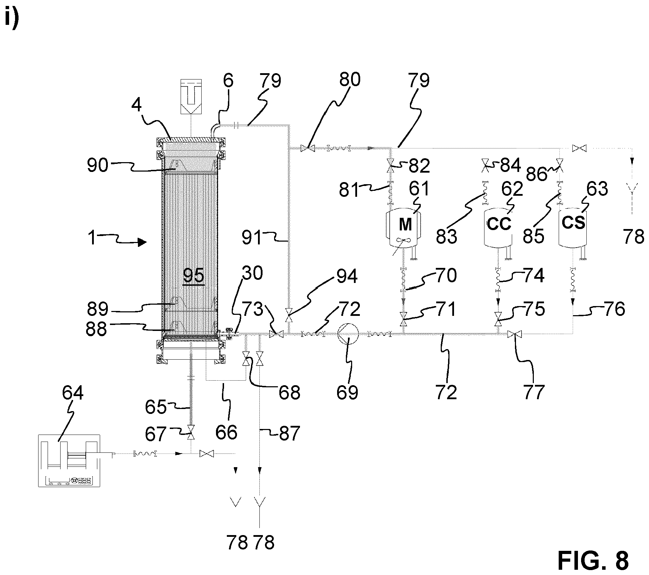

The sequence illustrated in **Fig. 8 a)****-i)** is used to explain the manufacturing method which is possible with a reactor 1 as described above.

In the context of the **Fig. 8 a)** the individual reference numerals are described, the same reference numerals are used in the following figures b)-i) and designate the same elements, but in the context of the following figures only the respective method step description is given.

**Fig. 8** schematically illustrates on the left side the reactor 1, and in this reactor 1 the mounting structure 23 and the attachment plate 22 is in each case illustrated in three different positions, the bottom position 88, the middle position 89, and the uppermost position 90. Depending on the corresponding process situation, only one of these positions is assumed.

There are three media containers, an actual growth medium container 61 (M), a container for calcium chloride 62 (CC) which acts as the hardening agent, and a container 63 (CS) for a cross-linking solution.

Also there is provided a container or a reservoir together with a pump for the actual cell paste, this is illustrated by reference numeral 64. This reservoir 64 is attached by way of valve 67 and via line 65 to the bottom inlet 25 of the reactor as illustrated above. To allow for complete filling of the space 92 below the attachment plate 22 in the first step of the manufacturing, which is illustrated in **Fig. 8 a)**, there is also provided an outlet 66 which can be opened and closed by way of valve 68 allowing air and/or paste to escape from the space 92 (see Fig. 1b).

The media container 61 is connected to the reactor by way of the line 70 controlled by way of the valve 71 leading to the collecting line 72. In this connecting line 72 there is a pump 69 and a valve 73 for control supply and/or removal from bottom inlet/outlet 30. Also there is a crossline 91 connecting this collecting line 72 with the upper branch of tubing, also here there is provided a corresponding valve 94.

Also connected to the collecting line 72 is the calcium chloride container 62 and this by way of line 74 which is controlled by valve 75. Also the container with the further crosslinking solution 63 is connected to collecting line by way of line 76 and controlled by valve 77.

In the upper branch there is an upper collecting line 79, which is attached to the second inlet/outlet 6 provided in the top cover 4 of the reactor. This upper collecting line 79 is connected by valve 80 and to the growth media container 61 by way recirculation line 81 controlled by way of valve 82, to the calcium chloride container 62 by way of circulation line 83 and to the further crosslinking solution container 63 by way of line 85 controlled by valve 86. The lines are also provided with means for outflow to collection means or waste means 78.

In **Fig. 8 a)** the step of prefilling with gel from the reservoir 64 is illustrated. In this step, the cell paste is pumped into the bioreactor. From reservoir 64, the paste is pumped through valve 67 and pipe 65 to the space 92 below the extrusion plate 24 and in this case the second upper extrusion plate is in the bottom position 88. The extrusion is carried on until the cell paste passes through both adjacent extrusion plates 22,24 or rather through the perforations in these extrusion plates 22,24 and forms a layer of paste above the attachment plate 22 or forms at least some widening portions. To allow a complete filling of the space 92 the line 66 is opened until the space is completely filled and outflow is then controlled by way of valve 68.

Once a layer (or widening portions) of gel above the attachment plate 22 is achieved, the next step as illustrated in **Fig. 8 b)** is initiated, the step of upper clamping. Now the container for calcium chloride 62 is connected, i.e. valves 75, 73 are opened and the pump 69 is activated such that the reactor volume is now filled with calcium chloride solution. This leads to a hardening of the cell paste layer above the second, upper extrusion plate and leads to the automatic adherence of individual strings of paste located in and above the nozzle openings. During this phase, the pumping of the cell paste is interrupted.

In the next step, schematically illustrated in **Fig. 8 c)**, the paste is extruded from container 64 and in parallel the mounting structure 23 is successively moved upwards until reaching the position as illustrated in this figure, i.e. at the end of the process the attachment plate 22 is in the position 90, that is the uppermost position. So in that phase the pumping of the cell paste is turned on again, so extrusion takes place, and the clamping plate, i.e. the attachment plate 22 with the attached uppermost parts of the paste above the plate 22 moves upwards, forming multiple individual fibres from each or the holes of the attachment plate 22. During extrusion, these nascent fibres are in the hardening bath, so they are successfully hardened concomitant to extrusion. The speed of extrusion is chosen to be somewhat lower than the speed of upwards motion of the attachment plate 22 so there is certain degree of stretching during extrusion.

Then follows the step as illustrated in **Fig. 8 d)**. In this step, the pump of unit 64 is stopped, and also of the motion of attachment plate 22, but now the hardening solution is supplied by way of lines 74, 72 and 66 so across valves 75, 73 and 68 to the space 92, so that paste located in that interspace is also cross-linked.

So this leads to a situation that the bottom part of the fibres is also clamped by way of this contiguous patch of paste located below the extrusion plate 24. So at the end of this process phase there is a bundle of microfibrillar fibres clamped by two contiguous patches of cross-linked or hardened paste, one above the attachment plate 22, and a second one below the extrusion plate 24.

This is then followed by what is illustrated in **Fig. 8 e)**, here a second crosslinking solution CS is introduced into the reaction cavity from container 63, for example an actual crosslinking solution acting on the protein contents of the paste. The calcium chloride solution is drained from the container during that step.

As illustrated in **Fig. 8 f)**, now the complete reaction container volume 94 is filled with the crosslinking solution from container 63, leading to a final crosslinking of the microfibrillar structure.

As illustrated in **Fig. 8 g)** the crosslinking is then drained from the reactor by opening valve 30 and allowing the crosslinking solution to drain by way of line 87, while at the same time by opening valves 71 and 94 and activating pump 69, culturing medium M is pumped from media container 61 by way of lines 70, 72, 91 and 79 into the container from the top.

This leads to the situation as illustrated in **Fig. 8 h)**, now the volume 94 of the reactor is filled with growth medium leading to growth of the cells in or on the fibrillary structures. If the paste does not already contain cells, during this step or before media can be supplied with cells, and corresponding seeding of the microfibrillar structure can take place.

This is the followed as illustrated in **Fig. 8 i)** by the actual cultivation process, so the reactor is kept at the appropriate temperature and cultivation conditions to lead to corresponding growth of muscle cells in an/or on the microfibrils. Optionally, the attachment plate 22 can move up and down in this phase to create a cyclic mechanical stimulation of the fibres. After this process is finished, the media can be allowed to flow out of the container and the microfibrillar structure can be taken out and can be further processed to lead to a cultivated meat product.

**LIST OF REFERENCE SIGNS**

| | | | |
|---|---|---|---|
| 1 | reactor | 20 | passage around 17 |
| 2 | circumferential wall of 1 | 21 | protruding portion of 19 |
| 3 | reinforcement structure | 22 | attachment plate |
| 4 | top cover | 23 | mounting structure for 22 |
| 5 | first inlet/outlet in 4 | 24 | extrusion plate |
| 6 | second inlet/outlet in 4 | 25 | bottom inlet |
| 7 | upper top closure bracket | 26 | Lower bottom closure bracket |
| 8 | lower top closure bracket | 27 | upper bottom closure bracket |
| 9 | hinge of 7 | 28 | closure mechanism of 26 |
| 10 | hinge of 8 | 29 | closure mechanism of 27 |
| 11 | upper closure extension | 30 | bottom inlet/outlet |
| 12 | closure mechanism of 7 | 31 | sealing around 23 |
| 13 | closure mechanism of 8 | 32 | central opening in 22 |
| 14 | sealing between 4 and 11 | 33 | surface area of extrusion plate without nozzle openings |
| 15 | upper frame portion | | |
| 16 | sealing between 11 and 15 | 34 | bottom plate |
| 17 | insert | 35 | side inlet in 25 |
| 18 | dead volume in 17 | 36 | sealing around 24 |
| 19 | vertical pipe through 17 | 37 | bottom part |
| 38 | upper bottom part | 70 | line from media container |
| 39 | sealing | 71 | valve and 70 |
| 40 | lateral opening in 22 | 72 | collecting line |
| 41 | surface area of extrusion plate without nozzle openings | 73 | valve and 72 |
| | | 74 | line from container for calcium chloride |
| 42 | perforations in 22 | | |
| 43 | lower frame portion | 75 | valve and 74 |
| 44 | perforations in 24 | 76 | line from container for cross-linking solution |
| 45 | shifting bracket | | |
| 46 | rail | 77 | valve in 76 |
| 47 | motor | 78 | collection |
| 48 | bottom mounting structure | 79 | upper collecting line |
| 49 | mounting structure for 45 | 80 | valve in 79 |
| 50 | housing | 81 | recirculation line to media container |
| 51 | carrier structure for 46 | | |
| 52 | leg | 82 | valve in 81 |
| 53 | shifting ring | 83 | recirculation line to container for calcium chloride |
| 54 | magnet of 53 | | |
| 55 | magnet of 23 | 84 | valve in 83 |
| 56 | glass housing | 85 | recirculation line to container for cross-linking solution |
| 57 | space between 22 and 24 | | |
| 58 | space above 22 | 86 | valve in 85 |
| 59 | circumferential rim | 87 | outlet line from 72 |
| 60 | attachment rib | 88 | bottom position of 22 |
| 61 | media container | 89 | middle position of 22 |
| 62 | container for calcium chloride | 90 | uppermost position of 22 |
| 63 | container for cross-linking | 91 | cross-line |
| | solution | 92 | space below 24 |
| 64 | reservoir and pump for cell paste | 93 | supply/control elements |
| | | 94 | valve |
| 65 | pipe from 64 to 1 | 95 | reactor volume |
| 66 | outlet from bottom area | | |
| 67 | valve in 65 | M | media |
| 68 | valve in 66 | CC | calcium chloride |
| 69 | pump | CS | further cross linking solution |

## Claims

1. Method for the manufacturing of an elongated structure for the fabrication of cultivated meat, wherein
(a) a paste is extruded through an extrusion plate (24) with at least one nozzle opening (44) and at least partly through an adjacent attachment plate (22), preferably into a space (58) downstream of the attachment plate (22),
(b) paste located in said attachment plate (22) and/or in said space (58) is at least partly hardened,
(c) said paste is continued to be extruded through said extrusion plate (24) while increasing the distance between said extrusion plate (24) and said attachment plate (22), under formation of said elongated structure between said plates (22,24).

2. Method according to claim 1, wherein the extrusion takes place at an ejection speed under the generation of a speed gradient downstream of the respective nozzle opening (44) in the extrusion plate (24) by way of a distancing speed between extrusion plate (24) and said attachment plate (22) larger than the ejection speed (Se) to form said elongated fibrous structure.

3. Method according to any of the preceding claims, wherein in at least one of step (b), step (c) and after step (c), the paste in said space (58) and/or in the interspace between the plates (22,24) is immersed in a hardening bath, preferably a hardening bath comprising divalent or polyvalent cations or an acidic bath or a bath with a temperature different from the gel paste, more preferably a hardening bath comprising divalent cations.

4. Method according to any of the preceding claims, wherein said extrusion plate (24) and/or said attachment plate (22) comprises at least 20, preferably at least 100, more preferably at least 200 or in the range of 250-1000 nozzle openings,
and/or the nozzle openings cover 5 - 90% or between 5% and 95% of the plate cross-section area, more preferably between 20% and 90% or 20 - 80%, most preferably between 40% and 70%,
and/or the nozzle openings are provided in said extrusion plate and/or said attachment plate with a nozzle density of 1 - 5000 nozzles/cm2, preferably in the range of 5 - 400 nozzles/cm2,
and/or the nozzle openings have a diameter or maximum extension in the lateral direction in case of non-circular openings in the range of between 10 to 5000 µm, more preferably 100-1500 µm,
and/or wherein the attachment plate is a mesh, a porous plate, or a plate with nozzle openings, preferably a plate with nozzle openings aligned to nozzle openings of the nozzle plate (42, 44).

5. Method according to any of the preceding claims, wherein extrusion in steps (b) and (c) takes place into a reaction container, and subsequent to step (c), if needed followed by a step of further hardening of the extruded elongated structure, the reaction container is filled with culturing growth media and the elongated structures are used for growing meat cells seeded onto said structures and/or already contained in said paste.

6. Method according to any of the preceding claims, wherein
(a) said paste is extruded through said extrusion plate (24) and an adjacent attachment plate (22) into a space (58) downstream of the attachment plate (22),
(b) stopping or slowing down said extrusion and hardening paste located in said space (58) by flooding said space downstream of the attachment plate (22) with a hardening bath, preferably a hardening bath comprising divalent or polyvalent cations or an acidic bath or a bath with a temperature different from the gel paste, preferably a bath of calcium chloride (CC), wherein preferably the paste forms a hardened gel volume that is immobilized in and/or on the attachment plate through geometric constraints,
(c) followed by continued extrusion of said paste through said extrusion plate (24) while increasing the distance between said extrusion plate and said attachment plate (22), preferably with a relative distancing speed larger than the extrusion speed from said extrusion plate (24) under formation of said elongated, preferably fibrous structure between said nozzle openings (42, 44),
(d) stopping said distancing and extrusion, and hardening the paste in the form of elongated structures, preferably fibers, in the space (57) between the two nozzle plates (22, 24), by flooding said space downstream of the attachment plate (22) with a hardening bath, preferably a hardening bath comprising divalent or polyvalent cations or an acidic bath or a protein cross-linking bath, preferably a bath of calcium chloride (CC), and, optionally, hardening the paste in a space (92) upstream of the extrusion plate (24),
(e) replacing what is in the space (57) between the plates with culturing media and using the elongated structures, preferably fibers, for growing meat cells seeded onto said elongated structures, preferably fibers, and/or already contained in said paste.

7. Method according to any of the preceding claims, wherein said paste is a paste for the fabrication of cultivated meat, comprising of consisting of the following components:
(A) at least one polysaccharide that can form a solidified gel by the action of divalent or polyvalent cations, thermal gelling, light-induced addition reaction or light induced condensation reaction, or a combination thereof, in a concentration in the paste in the range of 0.01-200 g per L of component (D);
(B) optionally one or more proteins, in a concentration in the paste in the range of 0-500 g per L of component (D), preferably said protein(s) assembles with the polysaccharide of component (A) via supramolecular or covalent interaction or a combination thereof
(C) cells selected from mammalian cells, fish cells, crustaceous cells or a combination thereof, in a concentration in the paste in the range of 0-300 billion cells per L of component (D);
(D) water or a water-based culturing medium;
(E) additives different from (A)-(D), selected from the group consisting of crosslinking kinetic modifier in a concentration in the paste in the range of 0-500 mM, flow modifier in a concentration in the paste in the range of 0-200 g per L of component (D), or a combination thereof,
and/or wherein said hardening bath comprises Ca2+, Mg2+, Fe2+ and Fe3+ or a combination thereof in water, preferably at a concentration in the range of 1-500 mM, more preferably between 20-100 mM,
and/or wherein the hardening bath is buffered at a pH ranging from 6 to 8, preferably by using HEPES buffer, wherein further preferably the HEPES buffer is used in a concentration comprised between 1-100mM,
and/or wherein either the hardening bath comprises a protein cross-linking agent or subsequent to step (b) the fibres are immersed in a protein cross-linking bath with such a cross-linking agent, wherein preferably the cross-linking agent is selected from the group consisting of transglutaminase, peroxidase, laccase, tyrosinase, lysyl oxidase, glutaraldehyde, genipin, citric acid, or a combination thereof, preferably transglutaminase, and wherein if transglutaminase is used, it is comprised in an amount of 1-2000 U per mL of crosslinking bath, and wherein further preferably cross-linking is performed at a temperature in the range of 20-45°C, preferably for a time span in the range of 10-120 min, more preferably 30-90 min.

8. Method according to any of the preceding claims, wherein during culturing the two nozzle plates are mechanically and/or electrically oscillated relative to each other in particular for stimulating and influencing the growth process in the anisotropic microfibrillar structures.

9. Method according to any of the preceding claims, wherein the extrusion in step (c) takes place with a drawing factor, defined as the ratio of the ejection speed to the distancing speed, of at least 1.1, preferably at least 1.5, most preferably at least 2.

10. Reactor for carrying out the method according to any of the preceding claims, wherein it comprises a closable reactor container (1) of elongated shape along a main axis with constant cross-section, preferably of cylindrical shape, a, preferably stationary, extrusion plate (24) and at least one attachment plate (22) movably mounted in said reactor container (1), elements for controlled supply of paste for extrusion thereof upstream of said extrusion plate (24) and elements (45, 46, 47, 49, 53) for controllably moving said extrusion and/or attachment plates (22) along the main axis, as well as means for supplying at least one of culturing media, hardening media, cross-linking media to and from the inside of the reactor container (1), preferably also to a space upstream of said extrusion plate (24).

11. Reactor according to claim 10, wherein the extrusion plate (24) is located at one end of said closable reactor container, and said attachment plate (22) is located adjacent to the extrusion plate (24), and wherein preferably the attachment plate (24) can be moved by said elements (45-47, 49, 53) in a contactless manner, preferably by magnetic attraction, wherein preferably to this end the attachment plate (24) is mounted in and/or on a mounting structure (23) with at least one magnet (55) and outside of the reactor container (1) there is a movable magnetic element, preferably in the form of a ring (53) with at least one magnet (54), which movable magnetic element can be moved automatically, preferably by motor (43).

12. Reactor according to any of the preceding claims 10-11, wherein said extrusion plate (24) and/or said attachment plate (22) comprise a plurality of nozzle openings (42, 44), preferably at least 20, more preferably at least 100, most preferably at least 200 or in the range of 250-1000 nozzle openings,
and/or the nozzle openings cover 5 - 90% or between 5% and 95% of the plate cross-section area, more preferably between 20% and 90% or 20 - 80%, most preferably between 40% and 70%,
and/or the nozzle openings are provided in said extrusion plate and/or said attachment plate with a nozzle density of 1 - 5000 nozzles/cm2, preferably in the range of 5 - 400 nozzles/cm2,
and/or the nozzle openings have a diameter or maximum extension in the lateral direction in case of non-circular openings in the range of between 10 to 5000 µm, more preferably 100-1500 µm,
and/or wherein said first extrusion plate (24) and/or said attachment plate (22) further comprises at least one opening (32, 33, 40, 41) for controlled supply of liquids through the respective plate.

13. Reactor according to any of the preceding claims 10-12, wherein it comprises a control, in particular for controlling at least one of the extrusion speed of the paste, the distancing speed between the extrusion and attachment plates (22), the supply of and removal of at least one of culturing media, cross-linking media, hardening media.

14. Use of a method according to any of the preceding claims 1-9 for the manufacturing of a consumer cultured meat product, preferably using the corresponding elongated structures, preferably after cultivation in the form of bundles, in combination with
(i) fat and or oil-based components including cultured fat, vegetable fat or animal fat based components and its derivatives, in particular in combination with separately cultured fat cells and/or separately cultured fat cell aggregates,
and/or (ii) structuring agents including hydrocolloids, and/or cellulose and its derivatives, and/or proteins derived from plant, animal, recombinant technology, cell cultivation;
and/or (iii) connective tissue components including animal derived connective tissue, cultured connective tissue, in particular in combination with cultured fibroblast and/or chondrocytes and/or separately cultured fibroblast and/or chondrocytes aggregates,
in particular to form fibre bundles compact bundle, like meat piece.

15. Cultured meat obtained using a method according to any of the preceding claims 1-9, and/or obtained using a reactor according to any of the preceding claims 10-13.
